# EUROPEAN PATENT SPECIFICATION

(11) **EP 1 878 419 B1**
(45) Date of publication and mention of the grant of the patent: **05.03.2014**
(21) Application number: 06014620.6
(22) Date of filing: 13.07.2006
(51) Int. Cl.: A61K 6/083, A61K 6/00

(54) **Total etch dental adhesive composition**
Total Etch - Dentalklebstoffzusammensetzung
Composition d'adhésif dentaire - Total Etch

(43) Date of publication of application: 16.01.2008
(73) Proprietor: Dentsply DeTrey GmbH, 78467 Konstanz (DE)
(72) Inventor: Klee, Joachim E., 78315 Radolfzell (DE); Lehmann, Uwe, 78467 Konstanz (DE); Berger, Franziska, 78050 Villingen (DE)
(74) Representative: Hartz, Nikolai

(56) References cited:
- WO-A-03/013444
- WO-A-03/035013
- US-A1- 2002 143 138

## Description

### Field of the invention

The present invention relates to a dental adhesive. In particular, the present invention relates to a dental adhesive for use in a total-etch adhesive bonding technique for sealants, orthodontic brackets, anterior composite resins, posterior composite resins, bonded dental silver amalgam, resin cementation with posts, all-metal, porcelain-metal, composite resin, and ceramic restorations, splinting, core foundations, and conservative treatment of a worn dentition.

### Background to the invention

The concept of total etch adhesion for enamel and dentin is known. The total etch technique relates to the simultaneous etching of enamel and dentin using a phosphoric acid gel in order to remove a smear layer and to make microporosities accessible for a subsequently applied dental adhesive composition. The acid is completely removed by subsequent air-water jet spray washing. The tubule apertures are sealed by the protective bonding agent layer with resin tags adhering to the tubule walls and the resin-impregnated dentin surface.

However, the total etch technique is problematic in that excessive drying of the etched tooth surface (especially dentin) may lead to inferior bonding properties. In order to avoid a separate etching step and the problems associated with subsequent acid removal and drying, self etching compositions were proposed. Self etching composition typically have an acidic pH of less than 2 and are capable of passing the smear layer. However, the self etching technique gives rise to further problems even when a self cure activator is used for curing. The dental cement is frequently inactivated at the interface to the dental adhesive so that bonding and curing cannot proceed efficiently.

It is the problem of the present invention to provide a dental adhesive composition for use in a total-etch adhesive bonding technique wherein the problems associated with excessive drying are avoided and wherein an improved adhesion in terms of shear bond strength to dentin and enamel is provided at a level of at least 15 MPa, and whereby the adhesion to enamel and the adhesion to dentin in terms of the shear bond strength are of similar magnitude.

### Disclosure of the invention

The present invention provides a dental adhesive composition having a pH of at least 5, which comprises an aqueous solution containing
(a) a polymerizable N-substituted alkyl acrylic or acrylic acid amide monomer;
(b) a water-soluble polymerizable carboxylic acid; and
(c) a water-soluble organic solvent.

The present invention is based on the recognition that an extremely strong bonding may be provided based on an aqueous solution capable of adjusting the moisture of the tooth surface provided that the composition contains components which are efficiently wetting the surface of the tooth at a pH of at least 5 for forming a polymerizable film. Given the presence of water in the composition, the presence of ester bonds in the composition which might hydrolyse during storage should be avoided.

### Disclosure of the preferred embodiments

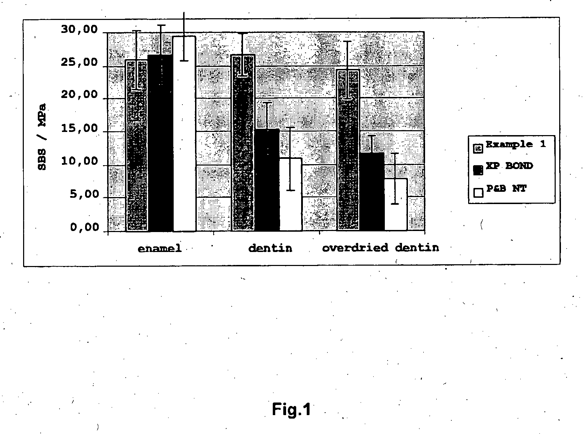

Figure 1 shows the adhesion between a composition according to Example 1 and a light curing composite (Spectrum TPH) in comparison to XP Bond and Prime&Bond NT in total etch technique, polymerized with a Spectrum 800 curing unit, whereby the storage conditions prior to shear bond strength measurement were storage for 24 h at 37°C and thermocyling between 5 to 55 °C for 1800 times.

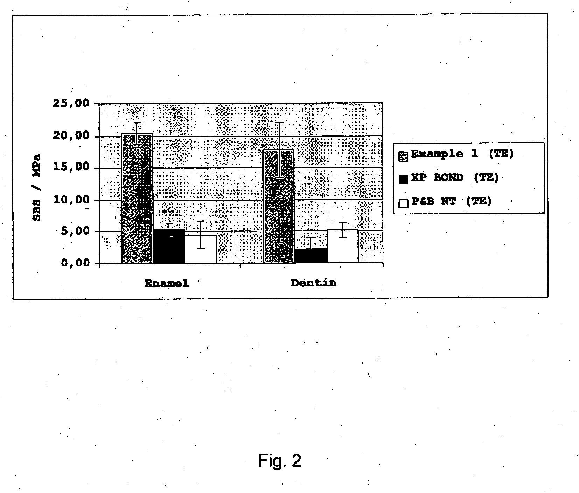

Figure 2 shows the adhesion between a composition according to Example 1 and a self-curing composite cement (Calibra) in a total etch technique while light curing the adhesive layer and self-curing the composite cement after 24 h/ 37°C, as compared to XP Bond and Prime&Bond NT in total etch technique.

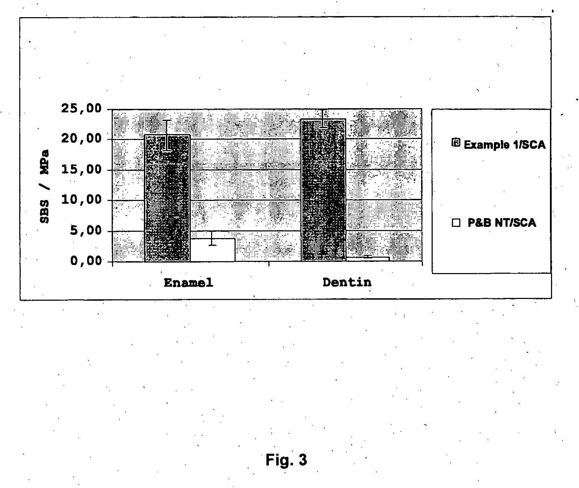

Figure 3 shows the adhesion between a mixture, comprising of a composition, according to Example 1 and a commercial self-cure activator (Prime&Bond Self-cure Activator), and a self-curing composite cement (Calibra) in a total etch technique while self-curing the adhesive layer and self-curing the composite cement after 24 h/ 37°C, as compared to XP Bond and Prime&Bond NT in total etch technique.

The dental adhesive composition according to the invention comprises an aqueous solution. The aqueous solution provides moisture for the tooth structure in case of excessive drying of the tooth surface in the total etch technique.

The aqueous solution contains an aqueous medium, polymerizable components and optionally an initiator, stabilizer and/or inhibitor. The dental adhesive composition has a pH of at least 5, preferably in the range of from 5 to 9.

The aqueous medium comprises water and optionally one or more water miscible organic solvents. Preferably, water is present in an amount of from 5 to 45 wt.% based on the dental adhesive composition. More preferably, water is present in an amount of from 20 to 40 wt.% based on the dental adhesive composition. If the water content is below 5 wt.%, then the dental adhesive might not be effective in moisturizing the tooth surface when the surface has been excessively dried.

The water-soluble organic solvent may be selected from alcohols and ketones. Specifically, the water-soluble organic solvent may be selected from ethanol, n-propanol, i-propanol, n-butanol, t-butanol, acetone, and methyl ethyl ketone. t-Butanol is particularly preferred. The water-soluble organic solvent may be present in an amount of from 5 to 45 wt.% based on the dental adhesive composition. More preferably, the water-soluble organic solvent may be present in an amount of from 8 to 30 wt.%.

The polymerizable components comprise a polymerizable N-substituted alkyl acrylic or acrylic acid amide monomer which may be preferably selected from compounds characterized by one of the following formulas: 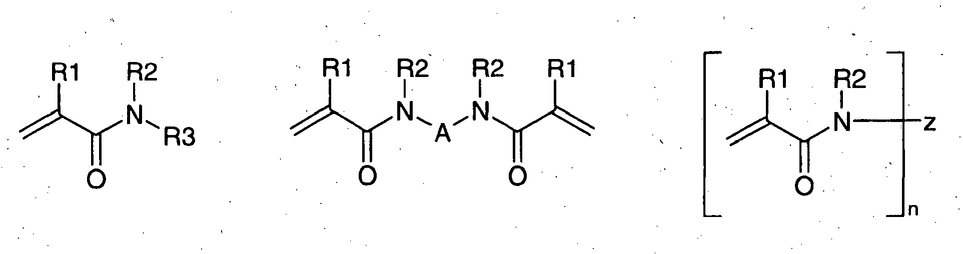 wherein R₁ and R₂ independently represent a hydrogen atom or a C₁ to C₈ alkyl group; A represents a divalent substituted or unsubstituted organic residue having from 1 to 11 carbon atoms, whereby said organic residue may contain from 1 to 3 oxygen and/or nitrogen atoms; Z represents a saturated at least trivalent substituted or unsubstituted C₁ to C₈ hydrocarbon group, a saturated at least trivalent substituted or unsubstituted cyclic C₃ to C₈ hydrocarbon group, and n is at least 3.

Preferably, the polymerizable monomer may be a mono-, bis- or poly(meth) acrylamide characterized by the following formula: 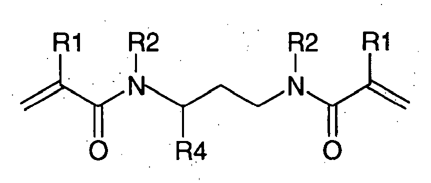 wherein R₁, R₂ and R₄ independently represent a hydrogen atom or a C₁ to C₈ alkyl group. Preferably, 1,3-Bisacrylamido propane (BAP) or 1,3-Bisacrylamido pentane (BAPEN) may be used.

The polymerizable water soluble N-substituted alkyl acrylic or acrylic acid amide monomer may be present in an amount of 20 to 60 wt.%, more preferably 30 to 50 wt.%, based on the dental adhesive composition.

Preferably, the polymerizable N-substituted alkyl acrylic or acrylic acid amide monomer has a molecular weight of at most 400, more preferably at most 300.

The polymerizable components also comprise a the water-soluble polymerizable carboxylic acid which may be selected from the group of mono- or polycarboxylic acids. Specifically, the mono- or polycarboxylic acids are selected from the group of acrylic acid, methacrylic acid, fumaric acid, maleic acid, itaconic acid, and mixtures thereof. The water-soluble organic acid may be present in an amount of from 3 to 20 wt.%, more preferably 5 to 15 wt.%, based on the dental adhesive composition.

Preferably, the water-soluble polymerizable carboxylic acid has a molecular weight of at most 400, more preferably at most 300.

Preferably, the polymerizable water soluble N-substituted alkyl acrylic or acrylic acid amide monomer and the water-soluble polymerizable carboxylic acid are contained in a ratio of from 7:1 to 1:1, preferably, 5:1 to 1:1 by weight.

The dental adhesive composition according to the present invention may further comprise a polymerization initiator, inhibitor and/or stabilizer. The polymerization initiator may be a thermal initiator, a redox-initiator or a photo initiator. The photo initiator may be camphor quinine/amine and/or an acylphosphine oxide. The inhibitor and/or stabilizer may be selected from hydroquinone, hydroquinone monomethyl ether, ditert. butyl cresol, tert. butyl hydroquinone.

In a specific embodiment, the dental adhesive composition may further comprise a nanofiller having an average particle size in the range of from 1 to 100 nm, preferably 1 to 10 nm.

In a specific embodiment, the dental adhesive composition may further comprise a fluoride containing compound.

In a preferred embodiment, the dental adhesive composition consists essentially of an aqueous solution containing
(a) a polymerizable N-substituted alkyl acrylic or acrylic acid amide monomer;
(b) a water-soluble polymerizable carboxylic acid; and
(c) a water-soluble organic solvent
and optionally a filler, fluoride containing compound, initiator, stabilizer and/or inhibitor.

Preferably, the dental adhesive composition provides adhesion in terms of shear bond strength to dentin and enamel at a level of at least 15 MPa, whereby the adhesion to dentin in terms of the shear bond strength is at least 50 %, more preferably at least 70 % of the adhesion to enamel in terms of the shear bond strength.

The invention will now be illustrated with reference to the following non-limiting examples

### Examples

### Example 1

The following dental adhesive composition was prepared. The composition had a pH of 5.5.

| Components/wt.% | Example 1 |
|---|---|
| Water | 28,800 |
| 1,3- Bisacrylamido propane | 23.047 |
| 1,3- Bisacrylamido pentane | 23.048 |
| Acrylic acid | 9.000 |
| t-Butanol | 12.713 |
| tert. Butyl hydroquinone | 0.051 |
| camphor quinone | 0.714 |
| Acylphosphin oxide (L-TPO Lucirin) | 1.796 |
| 4-Dimethylamino benzonitrile | 0.830 |
| TOTAL | 100 |

The mixture is a clear solution of low viscosity suitable for use in a total etch procedure. The composition provides excellent wetting properties of the tooth surface even when the tooth surface was overdried. Due to the absence of ester bonds, the storage stability of the composition is excellent.

### Example 2: Direct restoration

The composition according to Example 1 was applied to a dental surface and light cured. The dental surface was either enamel, dentin or overdried dentin. Prior to the application of the adhesive composition, the dental surface was etched with a conventional phosphoric acid gel.

A commercially available light curing composite (Spectrum TPH, shade 2, Dentsply) was applied to the light cured surface of the adhesive.

The shear bond strength was measured. The results were compared to the shear bond strength available by using commercially available adhesive composition XP Bond, Prime & Bond NT. The results are shown in Fig. 1

### Example 3: Indirect restoration

The composition according to Example 1 was applied to a dental surface and light cured. The dental surface was either enamel or dentin. Prior to the application of the adhesive composition, the dental surface was etched with a conventional phosphoric acid gel.

A commercially available self curing composite (Calibra) was applied to the light cured surface of the adhesive.

The shear bond strength was measured. The results were compared to the shear bond strength available by using commercially available adhesive composition XP Bond, Prime & Bond NT. The results are shown in Fig. 2.

A mixture of the composition of Example 1 and Prime&Bond self-cure activator was applied to a dental surface and self-cured. The dental surface was enamel or dentin. Prior to the application of the adhesive composition, the dental surface was etched with a conventional phosphoric acid gel.

A commercially available self curing composite (Calibra) was applied to the self-cured surface of the adhesive layer.

The shear bond strength was measured. The results were compared to the shear bond strength available by using commercially available adhesive compositions XP Bond, Prime & Bond NT. The results are shown in Fig. 3.

## Claims

1. A dental adhesive composition having a pH of at least 5 for use in a total-etch adhesive bonding technique, which comprises an aqueous solution containing
(a) a polymerizable N-substituted alkyl acrylic or acrylic acid amide monomer;
(b) a water-soluble polymerizable carboxylic acid; and
(c) a water-soluble organic solvent.

2. The dental adhesive composition for use in a total-etch adhesive bonding technique according to claim 1, wherein the polymerizable N-substituted alkyl acrylic or acrylic acid amide monomer is **characterized by** one of the following formulas: 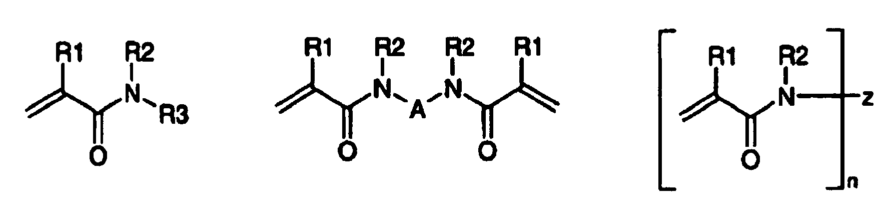 wherein
R¹ and R² independently represent
a hydrogen atom or
a C₁ to C₈ alkyl group,
A represents
a divalent substituted or unsubstituted organic residue having from 1 to 11 carbon atoms, whereby said organic residue may contain from 1 to 3 oxygen and/or nitrogen atoms,
Z represents
a saturated at least trivalent substituted or unsubstituted C₁ to C₈ hydrocarbon group, a saturated at least trivalent substituted or unsubstituted cyclic C₃ to C₈ hydrocarbon group, and
n is at least 3.

3. The dental adhesive composition for use in a total-etch adhesive bounding technique of claim 1 or 2, wherein the water-soluble polymerizable carboxylic acid is selected from the group of mono- or polycarboxylic acids.

4. The dental adhesive composition for use in a total-etch adhesive bounding technique of claim 3, wherein the mono- or polycarboxylic acids are selected from the group of acrylic acid, methacrylic acid, fumaric acid, maleic acid, citric acid, itaconic acid, formic acid and mixtures thereof.

5. The dental adhesive composition for use in a total-etch adhesive bounding technique according to any one of the preceding claims, wherein the water-soluble organic solvent is selected from alcohols and ketones.

6. The dental adhesive composition for use in a total-etch adhesive bonding technique according to claim 5, wherein the water-soluble organic solvent is selected from ethanol, n-propanol, i-propanol, n-butanol, t-butanol, acetone, and methyl ethyl ketone.

7. The dental adhesive composition for use in a total-etch adhesive bonding technique according to any one of the preceding claims, wherein the polymerizable water soluble N-substituted alkyl acrylic or acrylic acid amide monomer is present in an amount of 20 to 60 wt.%

8. The dental adhesive composition for use in a total-etch adhesive bonding technique according to any one of the preceding claims, wherein the water-soluble organic acid is present in an amount of from 3 to 20 wt.%.

9. The dental adhesive composition for use in a total-etch adhesive bonding technique according to any one of the preceding claims, wherein the water-soluble organic solvent, is present in an amount of from 5 to 45 wt. %

10. The dental adhesive composition for use in a total-etch adhesive bonding technique according to any one of the preceding claims, wherein water is present in an amount of from 5 to 45 wt.%.

11. The dental adhesive composition composition for use in a total-etch adhesive bonding technique according to any one of the preceding claims, wherein components (a) and (b) are contained in a ratio of from 7:1 to 1:1 by weight.

12. The dental adhesive composition for use in a total-etch adhesive bonding technique according to any one of the preceding claims, wherein said polymerizable monomer is a mono-, bis- or poly(meth) acrylamide **characterized by** the following formula: 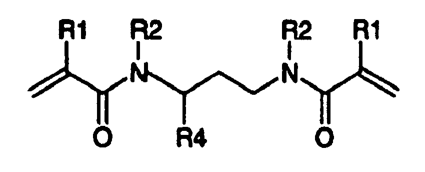 wherein
R¹, R² and R⁴ independently represent
a hydrogen atom or
a C₁ to C₈ alkyl group.

13. The dental adhesive composition for use in a total-etch adhesive bonding technique according to any one of the preceding claims, wherein said polymerizable N-substituted alkyl acrylic or acrylic acid amide monomer has a molecular weight of at most 400.

14. The dental adhesive composition for use in a total-etch adhesive bonding technique according to any one of the preceding claims, wherein said water-soluble polymerizable carboxylic acid has a molecular weight of at most 400.

15. The dental adhesive composition for use in a total-etch adhesive bounding technique according to any one of the preceding claims, which further comprises a polymerization initiator, inhibitor and/or stabilizer.

16. The dental adhesive composition for use in a total-etch adhesive bonding technique according to any one of the preceding claims, wherein said polymerization initiator is a thermal initiator, a redox-initiator or a photo initiator.

17. The dental adhesive composition for use in a total-etch adhesive bonding techniaue according to claim 14, wherein said photo initiator is acylphosphine oxide and/or camphor quinone / amine.

18. The dental adhesive composition for use in a total-etch adhesive bonding technique according to any one of the preceding claims, further comprising a nanofiller.

19. The dental adhesive composition for use in a total-etch adhesive bonding technique according to any one of the preceding claims, further comprising a fluoride compound.

## Patentansprüche

1. Dentaladhäsivzusammensetzung mit einem pH von mindestens 5 zur Verwendung in einer Total-Etch-Adhäsiv Bonding-Technik, die eine wässrige Lösung umfasst, die
(a) ein polymerisierbares N-substituiertes Alkylacryl- oder Acrylsäureamidmonomer;
(b) eine wasserlösliche polymerisierbare Carbonsäure; und
(c) ein wasserlösliches organisches Lösungsmittel
enthält.

2. Die Dentaladhäsivzusammensetzung zur Verwendung in einer Total-Etch-Adhäsiv Bonding-Technik nach Anspruch 1, wobei das polymerisierbare N-substituierte Alkylacryl- oder Acrylsäureamidmonomer **gekennzeichnet ist durch** eine der folgenden Formeln: 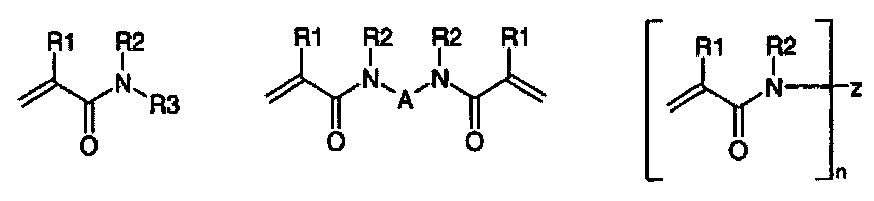 wobei
R¹ und R² unabhängig für ein Wasserstoffatom oder eine C₁ bis C₈ Alkylgruppe stehen,
A für einen zweiwertigen substituierten oder unsubstituierten organischen Rest mit 1 bis 11 Kohlenstoffatomen steht, wobei der organische Rest 1 bis 3 Sauerstoff- und/oder Stickstoffatome enthalten kann,
Z für eine gesättigte mindestens dreiwertige substituierte oder unsubstituierte C₁ bis C₈ Kohlenwasserstoffgruppe oder eine zyklische gesättigte mindestens dreiwertige substituierte oder unsubstituierte C₃ bis C₈ Kohlenwasserstoffgruppe steht und
n für mindestens 3 steht.

3. Die Dentaladhäsivzusammensetzung zur Verwendung in einer Total-Etch-Adhäsiv Bonding-Technik nach Anspruch 1 oder 2, wobei die wasserlösliche polymerisierbare Carbonsäure ausgewählt ist aus Mono- oder Polycarbonsäuren.

4. Die Dentaladhäsivzusammensetzung zur Verwendung in einer Total-Etch-Adhäsiv Bonding-Technik nach Anspruch 3, wobei die Mono- oder Polycarbonsäuren ausgewählt sind aus der Gruppe von Acrylsäure, Methacrylsäure, Fumarsäure, Maleinsäure, Zitronensäure, Itaconsäure, Ameisensäure und Mischungen daraus.

5. Die Dentaladhäsivzusammensetzung zur Verwendung in einer Total-Etch-Adhäsiv Bonding-Technik nach einem der vorstehenden Ansprüche, wobei das wasserlösliche organische Lösungsmittel ausgewählt wird aus Alkoholen und Ketonen.

6. Die Dentaladhäsivzusammensetzung zur Verwendung in einer Total-Etch-Adhäsiv Bonding-Technik nach Anspruch 5, wobei das wasserlösliche organische Lösungsmittel ausgewählt wird aus Ethanol, n-Propanol, i-Propanol, n-Butanol, t-Butanol, Aceton und Methylethylketon.

7. Die Dentaladhäsivzusammensetzung zur Verwendung in einer Total-Etch-Adhäsiv Bonding-Technik nach einem der vorstehenden Ansprüche, wobei das polymerisierbare wasserlösliche N-substituierte Alkylacryl- oder Acrylsäureamidmonomer in einer Menge von 20 bis 60 Gewichts-% vorliegt.

8. Die Dentaladhäsivzusammensetzung zur Verwendung in einer Total-Etch-Adhäsiv Bonding-Technik nach einem der vorstehenden Ansprüche, wobei die wasserlösliche organische Säure in einer Menge von 3 bis 20 Gewichts-% vorliegt.

9. Die Dentaladhäsivzusammensetzung zur Verwendung in einer Total-Etch-Adhäsiv Bonding-Technik nach einem der vorstehenden Ansprüche, wobei das wasserlösliche organische Lösungsmittel in einer Menge von 5 bis 45 Gewichts-% vorliegt.

10. Die Dentaladhäsivzusammensetzung zur Verwendung in einer Total-Etch-Adhäsiv Bonding-Technik nach einem der vorstehenden Ansprüche, wobei Wasser in einer Menge von 5 bis 45 Gewichts-% vorliegt.

11. Die Dentaladhäsivzusammensetzung zur Verwendung in einer Total-Etch-Adhäsiv Bonding-Technik nach einem der vorstehenden Ansprüche, wobei Komponten (a) und (b) enthalten sind in einem Gewichtsverhältnis von 7:1 bis 1:1.

12. Die Dentaladhäsivzusammensetzung zur Verwendung in einer Total-Etch Adhäsiv-Bonding-Technik nach einem der vorstehenden Ansprüche, wobei das polymerisierbare Monomer ein Mono-, Bis- oder Poly(meth)acrylamid ist, das **gekennzeichnet ist durch** die folgende Formel: 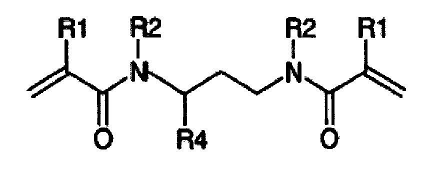 wobei
R¹, R² und R⁴ unabhängig für ein Wasserstoffatom oder eine C₁ bis C₈ Alkylgruppe stehen.

13. Die Dentaladhäsivzusammensetzung zur Verwendung in einer Total-Etch-Adhäsiv Bonding-Technik nach einem der vorstehenden Ansprüche, wobei das polymerisierbare N-substituierte Alkylacryl- oder Acrylsäureamidmonomer ein Molekulargewicht von höchstens 400 aufweist.

14. Die Dentaladhäsivzusammensetzung zur Verwendung in einer Total-Etch-Adhäsiv Bonding-Technik nach einem der vorstehenden Ansprüche, wobei die wasserlösliche polymerisierbare Carbonsäure ein Molekulargewicht von höchstens 400 aufweist.

15. Die Dentaladhäsivzusammensetzung zur Verwendung in einer Total-Etch-Adhäsiv Bonding-Technik nach einem der vorstehenden Ansprüche, die ferner einen Polymerisationsinitiator, Inhibitor und/oder Stabilisator umfasst.

16. Die Dentaladhäsivzusammensetzung zur Verwendung in einer Total-Etch-Adhäsiv Bonding-Technik nach einem der vorstehenden Ansprüche, wobei der Polymerisationsinitiator ein thermischer Initiator, ein Redox-Initiator oder eine Photoinitiator ist.

17. Die Dentaladhäsivzusammensetzung zur Verwendung in einer Total-Etch-Adhäsiv Bonding-Technik nach Anspruch 14, wobei der Photoinitiator Acylphosphinoxid und/oder Kampferchinon/Amin ist.

18. Die Dentaladhäsivzusammensetzung zur Verwendung in einer Total-Etch-Adhäsiv Bonding-Technik nach einem der vorstehenden Ansprüche, ferner umfassend einen Nanofüllstoff.

19. Die Dentaladhäsivzusammensetzung zur Verwendung in einer Total-Etch-Adhäsiv Bonding-Technik nach einem der vorstehenden Ansprüche, ferner umfassend eine Fluoridverbindung.

## Revendications

1. Composition d'adhésif dentaire ayant un pH d'au moins 5 pour une utilisation dans une technique de liaison adhésive par attaque chimique totale, qui comprend une solution aqueuse contenant :
(a) un monomère d'amide d'acide acrylique ou alkylacrylique N-substitué polymérisable ;
(b) un acide carboxylique polymérisable hydrosoluble ; et
(c) un solvant organique hydrosoluble.

2. Composition d'adhésif dentaire pour une utilisation dans une technique de liaison adhésive par attaque chimique totale suivant la revendication 1, dans laquelle le monomère d'amide d'acide acrylique ou alkylacrylique N-substitué polymérisable est **caractérisé par** une des formules suivantes : 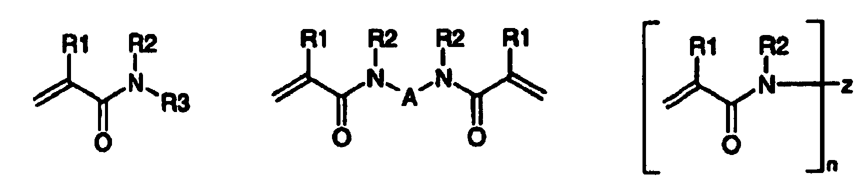 dans lesquelles :
R¹ et R² représentent indépendamment :
un atome d'hydrogène ou
un groupe alkyle en C₁ à C₈,
A représente :
un résidu organique divalent, substitué ou non substitué, ayant 1 à 11 atomes de carbone, ledit résidu organique pouvant contenir ainsi 1 à 3 atomes d'oxygène et/ou d'azote,
Z représente :
un groupe hydrocarboné en C₁ à C₈ saturé au moins trivalent, substitué ou non substitué, un groupe hydrocarboné en C₃ à C₈ cyclique saturé au moins trivalent, substitué ou non substitué, et
n est égal à au moins 3.

3. Composition d'adhésif dentaire pour une utilisation dans une technique de liaison adhésive par attaque chimique totale suivant la revendication 1 ou 2, dans laquelle l'acide carboxylique polymérisable hydrosoluble est choisi dans le groupe des acides mono- ou polycarboxyliques.

4. Composition d'adhésif dentaire pour une utilisation dans une technique de liaison adhésive par attaque chimique totale suivant la revendication 3, dans laquelle les acides mono- ou polycarboxyliques sont choisis dans le groupe comprenant l'acide acrylique, l'acide méthacrylique, l'acide fumarique, l'acide maléique, l'acide citrique, l'acide itaconique, l'acide formique et leurs mélanges.

5. Composition d'adhésif dentaire pour une utilisation dans une technique de liaison adhésive par attaque chimique totale suivant l'une quelconque des revendications précédentes, dans laquelle le solvant organique hydrosoluble est choisi entre des alcools et des cétones.

6. Composition d'adhésif dentaire pour une utilisation dans une technique de liaison adhésive par attaque chimique totale suivant la revendication 5, dans laquelle le solvant organique hydrosoluble est choisi entre l'éthanol, le n-propanol, l'isopropanol, le tertiobutanol, l'acétone et la méthyléthylcétone.

7. Composition d'adhésif dentaire pour une utilisation dans une technique de liaison adhésive par attaque chimique totale suivant l'une quelconque des revendications précédentes, dans laquelle le monomère d'amide d'acide acrylique ou alkylacrylique N-substitué hydrosoluble polymérisable est présent en une quantité de 20 à 60 % en poids.

8. Composition d'adhésif dentaire pour une utilisation dans une technique de liaison adhésive par attaque chimique totale suivant l'une quelconque des revendications précédentes, dans laquelle l'acide organique hydrosoluble est présent en une quantité de 3 à 20 % en poids.

9. Composition d'adhésif dentaire pour une utilisation dans une technique de liaison adhésive par attaque chimique totale suivant l'une quelconque des revendications précédentes, dans laquelle le solvant organique hydrosoluble est présent en une quantité de 5 à 45 % en poids.

10. Composition d'adhésif dentaire pour une utilisation dans une technique de liaison adhésive par attaque chimique totale suivant l'une quelconque des revendications précédentes, dans laquelle de l'eau est présente en une quantité de 5 à 45 % en poids.

11. Composition d'adhésif dentaire pour une utilisation dans une technique de liaison adhésive par attaque chimique totale suivant l'une quelconque des revendications précédentes, dans laquelle les constituants (a) et (b) sont présents en un rapport de 7:1 à 1:1 en poids.

12. Composition d'adhésif dentaire pour une utilisation dans une technique de liaison adhésive par attaque chimique totale suivant l'une quelconque des revendications précédentes, dans laquelle ledit monomère polymérisable est un mono-, bis- ou poly(méth)acrylamide **caractérisé par** la formule suivante : 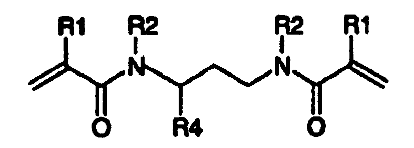 dans laquelle :
R¹, R² et R⁴ représentent indépendamment :
un atome d'hydrogène ou
un groupe alkyle en C₁ à C₈.

13. Composition d'adhésif dentaire pour une utilisation dans une technique de liaison adhésive par attaque chimique totale suivant l'une quelconque des revendications précédentes, dans laquelle ledit monomère d'amide d'acide acrylique ou alkylacrylique N-substitué polymérisable a un poids moléculaire d'au plus 400.

14. Composition d'adhésif dentaire pour une utilisation dans une technique de liaison adhésive par attaque chimique totale suivant l'une quelconque des revendications précédentes, dans laquelle ledit acide carboxylique polymérisable hydrosoluble a un poids moléculaire d'au plus 400.

15. Composition d'adhésif dentaire pour une utilisation dans une technique de liaison adhésive par attaque chimique totale suivant l'une quelconque des revendications précédentes, qui comprend en outre un initiateur de polymérisation, un inhibiteur et/ou un stabilisant.

16. Composition d'adhésif dentaire pour une utilisation dans une technique de liaison adhésive par attaque chimique totale suivant l'une quelconque des revendications précédentes, dans laquelle ledit initiateur de polymérisation est un initiateur thermique, un initiateur Redox ou un photo-initiateur.

17. Composition d'adhésif dentaire pour une utilisation dans une technique de liaison adhésive par attaque chimique totale suivant la revendication 14, dans laquelle ledit photo-initiateur est l'oxyde d'acylphosphine et/ou la camphoquinone/amine.

18. Composition d'adhésif dentaire pour une utilisation dans une technique de liaison adhésive par attaque chimique totale suivant l'une quelconque des revendications précédentes, comprenant en outre une nanocharge.

19. Composition d'adhésif dentaire pour une utilisation dans une technique de liaison adhésive par attaque chimique totale suivant l'une quelconque des revendications précédentes, comprenant en outre un fluorure.
